(19) Europäisches Patentamt
European Patent Office
Office européen des brevets

(11) Veröffentlichungsnummer: **0 538 689 A1**

(12) **EUROPÄISCHE PATENTANMELDUNG**

(21) Anmeldenummer: **92117260.7**

(22) Anmeldetag: **09.10.92**

(51) Int. Cl.[5]: **C07D 309/28**, C07F 7/18, A61K 31/35

(30) Priorität: **22.10.91 DE 4134757**

(43) Veröffentlichungstag der Anmeldung: **28.04.93 Patentblatt 93/17**

(84) Benannte Vertragsstaaten: **AT BE CH DE DK ES FR GB GR IT LI LU NL PT SE**

(71) Anmelder: **BAYER AG**
**W-5090 Leverkusen 1 Bayerwerk(DE)**

(72) Erfinder: **Mittendorf, Joachim, Dr.**
**Pahlkestrasse 3**
**W-5600 Wuppertal 1(DE)**
Erfinder: **Kunisch, Franz, Dr.**
**Zum Hahnenberg 20**
**W-5068 Odenthal-Glöbusch(DE)**
Erfinder: **Babczinski, Peter, Dr.**
**In der Lohrenbeck 11**
**W-5600 Wuppertal 1(DE)**
Erfinder: **Plempel, Manfred, Dr.**
**Zwengenbergerstrasse 3c**
**W-5657 Haan 1(DE)**

(54) **Neue substituierte 2,3-Dihydropyrane, Verfahren zu ihrer Herstellung und ihre Verwendung als Arzneimittel.**

(57) Die vorliegende Erfindung betrifft neue substituierte 2,3-Dihydropyrane der allgemeinen Formel (I)

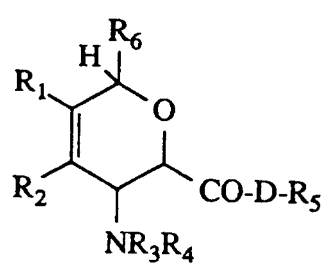

(I)

in welcher $R_6$, $R_1$, $R_2$, $R_3$, $R_4$, D und $R_5$ die in der Beschreibung angegebene Bedeutung haben, Verfahren zu ihrer Herstellung und ihre Verwendung als Arzneimittel, insbesondere als antimykotische Arzneimittel.

EP 0 538 689 A1

Die Erfindung betrifft neue substituierte 2,3-Dihydropyrane, Verfahren zu ihrer Herstellung und ihre Verwendung als Arzneimittel, insbesondere als antimykotische Arzneimittel.

In den Publikationen THL 1970, 293 und Tetrahedron 26 (1970), 3883; 3884 und 3900 sind die Methyl-4-amino-2,3,4-trideoxy-α-D-erythro-hex-2-enopyranosiduronicsäure und der entsprechende Methylester ohne pharmakologische Wirkung beschrieben.

Die Erfindung betrifft nun neue substituierte 2,3-Dihydropyrane der allgemeinen Formel (I)

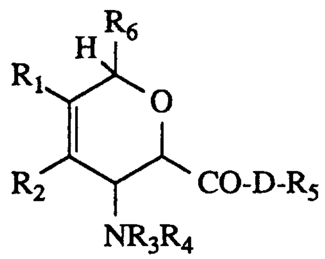

(I),

in welcher

| | |
|---|---|
| $R^6$ | für Wasserstoff oder geradkettiges oder verzweigtes Alkyl mit bis zu 8 Kohlenstoffatomen steht, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist, oder für eine Gruppe der Formel $-OSi(R)_3$ steht, worin |
| R | Methyl, Isopropyl, tert.-Butyl oder Phenyl bedeutet, |
| $R^1$ und $R^2$ | gleich oder verschieden sind und für Wasserstoff oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen substituiert ist, |
| $R^3$ | für eine Aminoschutzgruppe,<br>für Wasserstoff oder<br>für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,<br>oder<br>für geradkettiges oder verzweigtes Acyl oder Alkoxycarbonyl mit jeweils bis zu 8 Kohlenstoffatomen, oder<br>für Phenoxy oder Benzoyl steht, das gegebenenfalls wie oben beschrieben substituiert ist, oder<br>für eine Gruppe der Formel $-SO_2R^7$ steht,<br>worin |
| $R^7$ | geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^8R^9$ substituiert sind,<br>worin |
| $R^8$ und $R^9$ | gleich oder verschieden sind und Wasserstoff, geradkettiges oder verzweigtes Alkyl mit bis zu 8 Kohlenstoffatomen oder Phenyl bedeuten,<br>für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen |

oder durch eine Gruppe der Formel $-SO_2R^7$ oder $-NR^8R^9$ substituiert ist,
worin

$R^7$, $R^8$ und $R^9$ die oben angegebene Bedeutung haben,

$R^4$ für Wasserstoff oder für geradkettiges oder verzweigtes Alkyl mit bis zu 8 Kohlenstoffatomen steht, das gegebenenfalls durch Phenyl substituiert ist,

oder

$R^3$ und $R^4$ gemeinsam für den Rest der Formel $=CHR^{6'}$ stehen,
worin

$R^{6'}$ die oben angegebene Bedeutung von $R^6$ hat und mit dieser gleich oder verschieden ist,

D für ein Sauerstoff- oder Schwefelatom oder für die $>$NH-Gruppe steht,

$R^5$ für Wasserstoff oder für geradkettiges oder verzweigtes Alkyl mit bis zu 8 Kohlenstoffatomen, Benzyl oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-SO_2R^7$ oder $-NR^8R^9$ substituiert sind,
worin

$R^7$, $R^8$ und $R^9$ die oben angegebene Bedeutung haben,
oder
für geradkettiges oder verzweigtes Alkoxy mit bis zu 8 Kohlenstoffatomen steht, das gegebenenfalls durch geradkettiges oder verzweigtes Alkoxycarbonyl mit bis zu 6 Kohlenstoffatomen substituiert ist,

oder für den Fall, daß D für die $>$NH-Gruppe steht,

$R^5$ für die Gruppe der Formel $-SO_2R^7$ steht,
worin

$R^7$ die oben angegebene Bedeutung hat,

gegebenenfalls in einer isomeren Form, und deren Säureadditions-Salze und Metallsalzkomplexe.

Die erfindungsgemäßen Verbindungen können auch in Form ihrer Salze vorliegen. Im allgemeinen seien hier Salze mit organischen oder anorganischen Basen oder Säuren genannt.

Zu den Säuren, die addiert werden können, gehören vorzugsweise Halogenkohlenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoylcarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Bevorzugt werden Verbindungen der allgemeinen Formel (I), in welcher

$R^6$ für Wasserstoff oder geradkettiges oder verzweigtes Alkyl mit bis zu 6 Kohlenstoffatomen steht, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist, oder für eine Gruppe der Formel $-OSi(R)_3$ steht, worin

R Methyl, Isopropyl, tert.-Butyl oder Phenyl bedeutet,

$R^1$ und $R^2$ gleich oder verschieden sind und für Wasserstoff oder für geradkettiges oder verzweigtes Alkyl mit bis zu 6 Kohlenstoffatomen stehen, das gegebenenfalls durch Halogen, Hydroxy, durch geradkettiges oder verzweigtes Alkoxy, Acyl oder Alkoxycarbonyl mit jeweils bis zu 4 Kohlenstoffatomen substituiert ist,

$R^3$ für tert.Butyloxycarbonyl (BOC), Benzyloxycarbonyl (Z) oder Phenoxycarbonyl steht, oder für Wasserstoff oder für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind, oder für geradkettiges oder verzweigtes Acyl oder Alkoxycarbonyl mit jeweils bis zu 6 Kohlenstoffatomen oder für Phenoxy oder Benzoyl steht, das gegebenenfalls wie oben beschrieben substituiert ist, oder für eine Gruppe der Formel $-SO_2R^7$ steht, worin

$R^7$ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^8R^9$ substituiert ist, worin

$R^8$ und $R^9$ gleich oder verschieden sind und Wasserstoff, geradkettiges oder verzweigtes Alkyl mit bis zu 6 Kohlenstoffatomen bedeuten, für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-SO_2R^7$ oder $-NR^8R^9$ substituiert ist, worin

$R^7$, $R^8$ und $R^9$ die oben angegebene Bedeutung haben,

$R^4$ für Wasserstoff oder für geradkettiges oder verzweigtes Alkyl mit bis zu 6 Kohlenstoffatomen oder Benzyl steht,

oder

$R^3$ und $R^4$ gemeinsam für den Rest der Formel $=CHR^{6'}$ stehen, worin

$R^{6'}$ die oben angegebene Bedeutung von $R^5$ hat und mit dieser gleich oder verschieden ist,

D für ein Sauerstoff- oder Schwefelatom oder für die $>$NH-Gruppe steht,

$R^5$ für Wasserstoff oder für geradkettiges oder verzweigtes Alkyl mit bis zu 6 Kohlenstoffatomen, Benzyl oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit

jeweils bis zu 4 Kohlenstoffatomen oder durch eine Gruppe der Formel -$SO_2R^7$ oder -$NR^8R^9$ substituiert sind,
worin
$R^7$, $R^8$ und $R^9$ die oben angegebene Bedeutung haben,
oder
für geradkettiges oder verzweigtes Alkoxy mit bis zu 6 Kohlenstoffatomen steht, das gegebenenfalls durch geradkettiges oder verzweigtes Alkoxycarbonyl mit bis zu 4 Kohlenstoffatomen substituiert ist,

oder für den Fall, daß D für die $>$NH-Gruppe steht,
$R^5$ für die Gruppe der Formel -$SO_2R^7$ steht,
worin
$R^7$ die oben angegebene Bedeutung hat,

gegebenenfalls in einer isomeren Form und deren Säureadditions-Salze und Metallsalzkomplexe.

Besonders bevorzugt werden Verbindungen der allgemeinen Formel (I),
in welcher

$R^6$ für Wasserstoff oder geradkettiges oder verzweigtes Alkyl mit bis zu 4 Kohlenstoffatomen steht, oder
für eine Gruppe der Formel -$OSi(R)_3$ steht,
worin
R Methyl, Isopropyl, tert.-Butyl oder Phenyl bedeutet,
$R^1$ und $R^2$ gleich oder verschieden sind und
für Wasserstoff oder
für geradkettiges oder verzweigtes Alkyl mit bis zu 4 Kohlenstoffatomen stehen,
$R^3$ für tert.Butyloxycarbonyl (Boc), Benzyloxycarbonyl (Z) oder Phenoxycarbonyl steht, oder
für Wasserstoff steht, oder
für geradkettiges oder verzweigtes Alkyl mit bis zu 4 Kohlenstoffatomen steht, oder
für geradkettiges oder verzweigtes Acyl oder Alkoxycarbonyl mit jeweils bis zu 4 Kohlenstoffatomen oder für Phenoxy oder Benzoyl steht, oder
für eine Gruppe der Formel -$SO_2R^7$ steht,
worin
$R^7$ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
$R^4$ für Wasserstoff oder
für geradkettiges oder verzweigtes Alkyl mit bis zu 4 Kohlenstoffatomen steht,

oder
$R^3$ und $R^4$ gemeinsam für den Rest der Formel $=CHR^{6'}$ stehen,
worin
$R^{6'}$ die oben angegebene Bedeutung von $R^6$ hat und mit dieser gleich oder verschieden ist,
D für ein Sauerstoff- oder ein Schwefelatom oder für die $>$NH-Gruppe steht,
$R^5$ für Wasserstoff oder für geradkettiges oder verzweigtes Alkyl mit bis zu 4 Kohlenstoffatomen, Benzyl oder Phenyl steht, wobei letztere gegebenenfalls durch Fluor, Chlor, Brom, Nitro, Cyano, Methoxy oder Ethoxy oder durch eine Gruppe der Formel -$SO_2R^7$ oder -$NR^8R^9$ substituiert sind,
worin
$R^7$ die oben angegebene Bedeutung hat,
und
$R^8$ und $R^9$ gleich oder verschieden sind und Wasserstoff, Methyl oder Ethyl bedeuten, oder
für geradkettiges oder verzweigtes Alkoxy mit bis zu 4 Kohlenstoffatomen stehen, das gegebenenfalls durch geradkettiges oder verzweigtes Alkoxycarbonyl mit bis zu 4 Kohlenstoffatomen substituiert ist,

oder im Fall, daß D für die $>$NH-Gruppe steht,
$R^5$ für die Gruppe der Formel -$SO_2R^7$ steht,
worin
$R^7$ die oben angegebene Bedeutung hat,

gegebenenfalls in einer isomeren Form, und deren Säureadditions-Salze und Metallsalzkomplexe.

Ganz besonders bevorzugt sind die Verbindungen der allgemeinen Formel (I), in welcher die Reste der Formel -$NR^3R^4$ und -CO-D-$R^5$ in cis-Stellung vorliegen.

Außerdem wurde ein Verfahren zur Herstellung der erfindungsgemäßen Verbindungen der allgemeinen Formel (I) gefunden, dadurch gekennzeichnet, daß man

[A] Verbindungen der allgemeinen Formel (II)

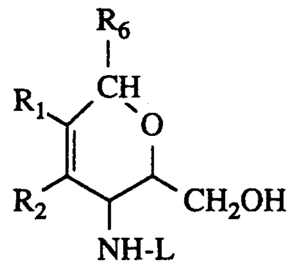

(II),

in welcher

$R^1$, $R^2$ und $R^6$ die oben angegebene Bedeutung haben,

und

L für eine der oben aufgeführten Aminoschutzgruppen, vorzugsweise für tert.Butyloxycarbonyl (Boc), steht,

in Anwesenheit von Oxidationsmitteln, gegebenenfalls in inerten Lösemitteln, zunächst zu den Verbindungen der allgemeinen Formel (III)

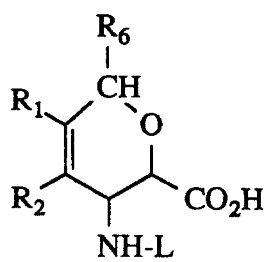

(III),

in welcher

$R^1$, $R^2$, $R^6$ und L die oben angegebene Bedeutung haben,

oxidiert

und anschließend gegebenenfalls die Säuren in inerten Lösemitteln entweder direkt, beispielsweise mit Diazomethan oder nach vorgeschalteter Aktivierung, gegebenenfalls in Anwesenheit von Hilfsmitteln und/oder einer Base, verestert

und gegebenenfalls unter Freisetzung der Aminfunktion ($R^3$,$R^4$ = H) die Aminoschutzgruppe L nach üblichen Methoden, vorzugsweise mit Säuren, zunächst abspaltet,

und im Fall, der für $R^3$, $R^4$, D und $R^5$ oben aufgeführten anderen Definitionen, nach üblichen Verfahren, wie beispielsweise Amidierung, Sulfonierung oder Sulfoamidierung, gegebenenfalls in Anwesenheit von Hilfsstoffen, wie Katalysatoren und/oder Dehydratisierungsmitteln, derivatisiert,

oder daß man

[B] Verbindungen der allgemeinen Formel (IV)

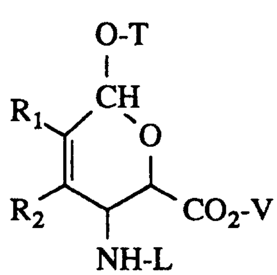

(IV),

in welcher

$R^1$, $R^2$ und L die oben angegebene Bedeutung haben,

T für eine typische Hydroxyschutzgruppe, vorzugsweise für tert.-Butyldimethylsilyl, steht,

und

V für eine Schutzgruppe, vorzugsweise p-Nitrobenzyl, steht,

umsetzt,

in einem nächsten Schritt die Schutzgruppe (V) in inerten Lösemitteln nach üblicher Methode, vorzugsweise mit Natriummethanolat abspaltet, anschließend die Gruppe O-T durch Umsetzung mit Acetanhydrid, $n\text{-}Bu_4N^{\oplus}F^{\ominus}$ in Pyridin in die $-OCO\text{-}CH_3$-Gruppe überführt und in einem letzten Schritt, ebenfalls nach üblicher Methode, vorzugsweise mit Triethylsilan in Anwesenheit von Trimethylsilyltriflat die Gruppe -O-T abspaltet, und im Fall, daß $R^6 \neq H$ eine Alkylierung anschließt,

oder gegebenenfalls Derivatisierungen sowohl an der Ester- als auch an der Aminfunktion, wie unter [A] beschrieben, durchführt,

und im Fall der Säuren (D = O, $R^5$ = H) die entsprechenden Ester verseift,

und im Fall der reinen Enantiomere, entweder die reinen D- oder L-Glucosederivate einsetzt, oder die Enantiomere

nach Derivatisierung als Diastereomere nach üblichen Methoden, beispielsweise durch fraktionierte Kristallisation, trennt.

Die erfindungsgemäßen Verfahren können durch folgendes Formelschema beispielhaft erläutert werden:

[A]

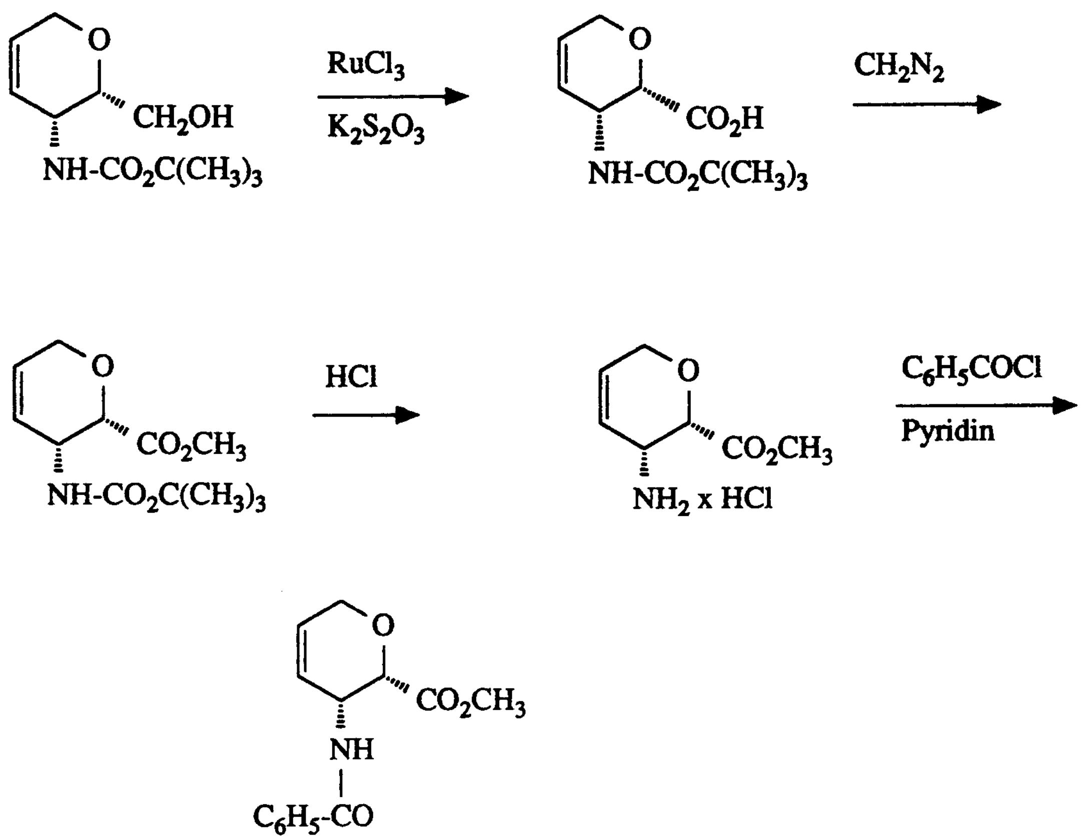


[B]

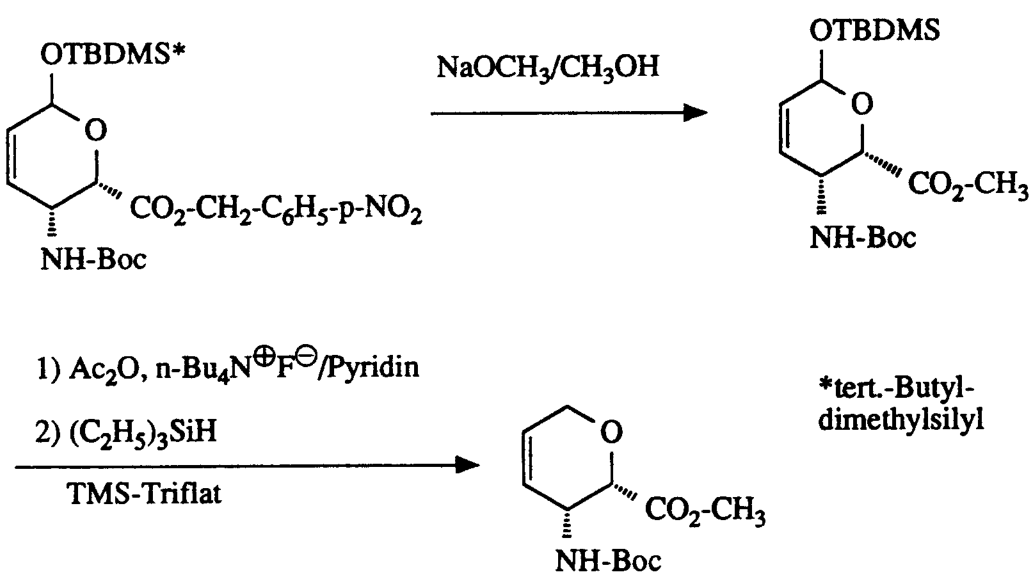


Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.-Butyl-dimethylsilyl, tert.-Butyldiphenylsilyl, Triphenylsilyl, Trimethylsilylethoxycarbonyl, Benzyl, Triphenylmethyl (Trityl), Monomethoxytrityl (MMTr), Dimethoxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert.-Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)-ethoxy]methyl, 2-(Methylthiomethoxy)-ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl.

Als Lösemittel für die grundlegenden Schritte der Verfahren kommen gegebenenfalls Wasser oder alle inerten organischen Lösemittel in Frage, die sich unter den Reaktionsbedingungen nicht verändern. Hierzu gehören vorzugsweise Alkohole wie Methanol, Ethanol, Propanol, Isopropanol, tert.Butanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind Dioxan, Diethylether, Tetrahydrofuran und tert.Butanol.

Die Reaktionstemperaturen können während der einzelnen Schritte in einem größeren Breich variiert werden. Im allgemeinen arbeitet man zwischen -30 °C und +150 °C, vorzugsweise zwischen -10 °C und +100 °C. Insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Als Oxidationsmittel eignen sich beispielsweise Rutheniumtrichlorid, Chrom-VI-oxid/Schwefelsäure/Aceton, Pyridiniumdichromat oder Pyridiniumchlorochromat.

Als Basen für die erfindungsgemäßen Verfahren können anorganische oder organisch Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl-($C_1$-$C_6$)amine) wie Triethylamin, oder Heterocyclen wie Pyridin, Methylpiperidin, Piperidin, Morpholin, 4-Dimethylaminopyridin oder 1,5-Diazabicyclo[5.4.0]-undec-5-en.

Als Säuren werden im allgemeinen Mineralsäuren wie beispielsweise Chlorwasserstoffsäure, Bromwasserstoffsäure, Schwefelsäure, Phosphorsäure oder aber Gemische der genannten Säuren, bevorzugt Chlorwasserstoffsäure eingesetzt.

Die Säure wird im allgemeinen in einer Menge von 2 mol bis 30 mol, bevorzugt von 5 mol bis 15 mol, bezogen auf 1 mol der Verbindungen der allgemeinen Formel (III), eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Die Verseifung der Carbonsäureester kann ebenso mit einer der oben aufgeführten Säuren durchgeführt werden.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in

üblicher Weise isoliert werden.

Die Abspaltung der Aminoschutzgruppe erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Abspaltung der Hydroxy- und Carboxyschutzgruppen erfolgt nach üblicher Methode, im Fall der Benzylgruppe beispielsweise in einem oben aufgeführten Lösemittel durch hydrogenolytische Spaltung in Anwesenheit eines Katalysators mit Greene: "Protective Groups in Organic Synthesis", J. Wiley and Sons, 1981, New York.

Beispielhaft für die oben aufgeführten Derivatisierungsmöglichkeiten sollen hier die Amidierung, Sulfonierung bzw. Sulfonamidierung und Alkylierung erläutert werden.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0 °C bis 150 °C, bevorzugt bei 25 °C bis 40 °C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexyfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int. Pept. Prot. Res. 13, 403 (1979), 17, 187 (1981)].

Die Sulfonierung oder Sulfoamidierung erfolgt in den oben erwähnten inerten Lösemitteln, gegebenenfalls unter Einsatz der ebenfalls oben aufgeführten Basen und Dehydratisierungsmittel.

Die Sulfonierung und Sulfoamidierung werden im allgemeinen bei Normaldruck durchgeführt. Es ist aber auch möglich, die Verfahren bei Unterdruck oder Überdruck durchzuführen (z.B. in einem Bereich von 0,5 bis 5 bar).

Die Sulfonierung und die Sulfoamidierung wird im allgemeinen in einem Temperaturbereich von 0 °C bis +150 °C, bevorzugt von +25 °C bis +40 °C, durchgeführt.

Zur Amidierung eignen sich im allgemeinen die literaturbekannten, käuflichen Amine und deren Derivate [vgl. Houben-Weyl, "Methoden der organischen Chemie", Band XI/1 und XI/2].

Die Sulfonierung und Sulfoamidierung erfolgen im allgemeinen ebenfalls mit den üblichen Sulfonsäuren und deren aktivierte Derivate [vgl. Houben-Weyl, "Methoden der organischen Chemie", Band IX, S 407 ff; Beilstein 11, 26].

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator, bezogen auf 1 mol Reaktionspartner, ein.

Sowohl die Veresterung als auch die Amidierung können gegebenenfalls über aktivierte Stufen der Carbonsäuren, wie beispielsweise Säurehalogenide, die aus der entsprechenden Säure durch Umsetzung

mit Thionylchlorid, Phosphortrichlorid, Phosphorpentachlorid, Phosphortribromid oder Oxalylchlorid hergestellt werden können, verlaufen.

Die Alkylierung wird in den oben aufgeführten Lösemitteln bei Temperaturen von 0 °C bis +150 °C, vorzugsweise bei Raumtemperatur bis +100 °C bei Normaldruck durchgeführt.

Als Alkylierungsmittel bei den Verfahren können beispielsweise ($C_1$-$C_8$)-Alkylhalogenide, Sulfonsäureester oder substituierte oder unsubstituierte ($C_1$-$C_6$)-Dialkyl- oder ($C_1$-$C_6$)-Diarylsulfate, vorzugsweise Methyljodid, p-Toluolsulfonsäureester oder Dimethylsulfat eingesetzt werden.

Die Säureadditions-Salze der Verbindungen der Formel (I) können in einfacher Weise nach üblichen Salzbildungsmethoden, z.B. durch Lösen einer Verbindung der Formel (I) in einem geeigneten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Verbindungen der allgemeinen Formel (II) sind neu und können hergestellt werden, indem man Verbindungen der allgemeinen Formel (V)

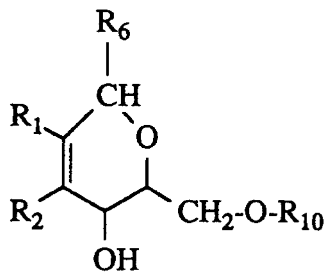

(V),

in welcher

$R^1$, $R^2$ und $R^6$ die oben angegebene Bedeutung haben,

und

$R^{10}$ für eine der oben aufgeführte Hydroxyschutzgruppen, vorzugsweise Dimethyl-tert.butyl-silyl, steht,

zunächst in einem der oben aufgeführten Lösemitteln, vorzugsweise Tetrahydrofuran, durch Umsetzung mit Diethylazodicarboxylat, Triphenylphosphin und $HN_3$ in die Verbindungen der allgemeinen Formel (VI),

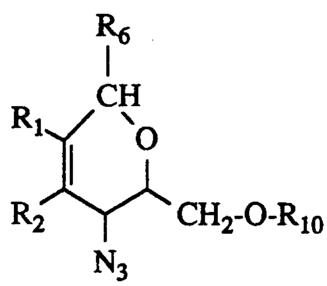

(VI),

in welcher

$R^1$, $R^2$, $R^6$ und $R^{10}$ die oben angegebene Bedeutung haben,

überführt, in einem weiteren Schritt zunächst mit Triphenylphosphin/Wasser in Tetrahydrofuran umsetzt und anschließend die Aminoschutzgruppe L nach üblicher Methode, in Anwesenheit einer Base, vorzugsweise Triethylamin einführt, und die Silylgruppe ebenfalls nach bekannten Methoden, beispielsweise mit Tetrabutylammoniumfluorid in einem der oben aufgeführten Lösemitteln, vorzugsweise Tetrahydrofuran abspaltet.

Die Verbindungen der allgemeinen Formel (V) sind größtenteils bekannt oder können nach üblicher Methode hergestellt werden [z.B. Angew. Chem. 102 (3), Angew.Chem.; Int. Ed. Engl. 1991 30 (3), 299-303].

Die Verbindungen der allgemeinen Formel (VI) sind neu und können beispielsweise nach dem oben aufgeführten Verfahren hergestellt werden.

Die Verbindungen der allgemeinen Formel (III) sind neu und können nach der unter [A] angegebenen Methode hergestellt werden.

Die Verbindungen der allgemeinen Formel (IV) sind teilweise bekannt (vgl. THL, Vol. 24, Nr. 43, S. 4661-4664, 1983) oder neu und können dann in Analogie zu der in der oben angegebenen Publikation aufgeführten Methode hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Verbindungen der allgemeinen Formel (I) und ihre Säureadditions-Salze weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden: Alle Dermatomykosen und Systemmykosen, insbesondere solche, die durch die oben genannten Erreger hervorgerufen werden.

Die erfindungsgemäßen Verbindungen wurden in den Applikationsarten i.v., s.c. und orale Gabe am Modell der Mäuse-Candidose auf ihre antimykotische in vivo-Wirksamkeit geprüft:
Männliche $CF_1$-SPF-Mäuse wurden mit 1-3 x $10^6$ Sprosszellen von C. albicans pro Tier durch Injektion der Keimsuspension in phys. NaCl-Lösung (0,2 ml/Tier) in die Schwanzvene infiziert. Nicht behandelte Kontrolltiere entwickeln unter diesen Infektionsbedingungen eine Nierencandidose und sterben zu 95-100% der eingesetzten Tiere innerhalb 6 Tagen p.i. an dieser Infektion. Wurden infizierte Tiere täglich 2 mal, beginnend mit dem Tag der Infektion, mit den erfindungsgemäßen Verbindungen oral oder parenteral in Dosen 2 x 25 bis 2 x 50 mg/kg Körpergewicht über 2 - 5 Tage behandelt, so überleben 60-90% der Tiere die Infektion in gutem Zustand. Die C.albicans-Keimzahlen in den Nieren der infizierten und behandelten Tiere liegen am 4. Tag p.i. durchschnittlich um 2 Zehnerpotenzen unter denen von unbehandelten, infizierten Kontrolltieren.

Tabelle A

| Bsp.Nr. | Dosis (mg/kg/d) | App. | Zahl der überl. Tiere |
|---|---|---|---|
| Kontrolle | | | 1/10 |
| 1 | 2 × 50 | po | 7/10 |
| 17 | 2 × 25 | sc | 3/10 |
| VII | 2 × 50 | po | 8/10 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermittln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Ausgangsverbindungen

Beispiel I

(2R,3S)-2,3-Dihydro-3-hydroxy-2-hydroxymethyl-6H-pyran

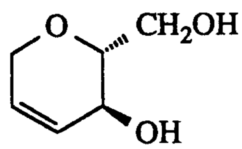

Eine Lösung von 4,6-Di-O-acetyl-1,5-anhydro-2,3-dideoxy-D-erythro-hex-2-enitol (77,0 g, 0,36 mol) und Natriummethanolat (6,6 g, 0,12 mol)in 950 ml Methanol wird 3 h bei Raumtemperatur gerührt. Die Lösung wird mit Dowex W x 50 ($H^+$-Form) neutralisiert, filtriert und im Vakuum eingeengt. Der Rückstand wird an Kieselgel chromatographiert (Essigester/Petrolether 2:3).
Ausbeute: 45,8 g (98% der Theorie)
$C_6H_{10}O_3$(130,1)
$^1$H-NMR ($CDCl_3$): $\delta$ = 3.31 (cm, 1H), 3.74 (cm, 1H), 3.83 (cm, 2H), 5.77, 5.82 (AB, $J_{AB}$ = 10Hz, 2H)

Beispiel II

(2S,3S)-2-[(tert.Butyldimethylsilyl)oxy]methyl-2,3-dihydro-3-hydroxy-6H-pyran

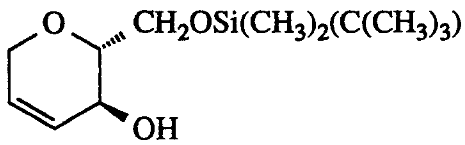

Zur Lösung der Verbindung aus Beispiel I (45,8 g, 0,352 mol), tert.Butyldimethylsilylchlorid (58,0 g, 0,385 mol) und 4-Dimethylaminopyridin (0,2 g) in 750 ml Dichlormethan wird unter Eiskühlung Triethylamin (39,1 g, 0,386 mol) getropft. Die Lösung wird 17 h bei Raumtemperatur gerührt, anschließend mit gesättigter wäßriger $NaHCO_3$-Lösung gewaschen, über $Na_2SO_4$ getrocknet, filtriert und im Vakuum eingeengt. Der Rückstand wird an Kieselgel chromatographiert (Essigester/Petrolether 2:3).
Ausbeute: 68,5 g (78% der Theorie)
$C_{12}H_{24}O_3Si$ (244,2)
$^1$H-NMR ($CDCl_3$): $\delta$ = 0.05 (s, 6H), 0.85 (s, 9H), 3.35 (d, J = 3Hz, 1H), 4.03 (s, 2H), 4.05 (cm, 1H), 5.67, 5.72 (AB, $J_{AB}$ = 10Hz, 2H)

Beispiel III

(2S,3R)-3-Azido-2-[(tert.butyldimethylsilyl)oxy]methyl-2,3-dihydro-6H-pyran

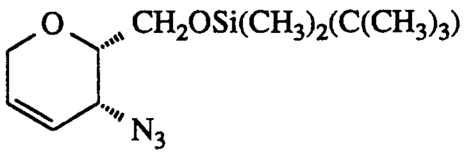

Diethylazodicarboxylat (49,2 g, 0,282 mol) wird tropfenweise unter Eiskühlung zu einer Lösung der Verbindung aus Beispiel II (66,4 g, 0,272 mol), Triphenylphosphin (85,7 g, 0,327 mol) und $HN_3$ (14,0g, 0,326 mol, 0,6 N Lösung in Toluol) in 2300 ml THF gegeben. Die Lösung wird 20 h bei Raumtemperatur gerührt, anschließend im Vakuum auf ein Volumen von ca. 400 ml eingeengt und filtriert. Das Filtrat wird im Vakuum eingeengt und der Rückstand an Kieselgel chromatographiert (Essigester/Petrolether 20:1)
Ausbeute: 44,5 g (61% der Theorie)
$C_{12}H_{23}N_3O_2Si$ (269,2)
$^{13}$C-NMR($CDCl_3$): $\delta$ = -5.44, -5.35, 18.30, 25.89, 53.30, 62.72, 66.33, 77.78, 121.27, 132.31

Beispiel IV

(2S,3R)-2-[(tert.Butyldimethylsilyl)oxy]methyl-3-(N-tert.butyloxycarbonyl)amino-2,3-dihydro-6H-pyran

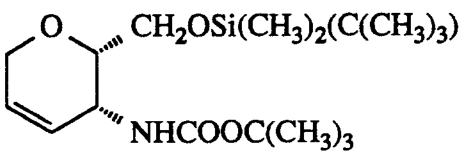

Eine Lösung der Verbindung aus Beispiel III (44,0 g, 0,163 mol) in 480 ml THF wird mit Triphenylphosphin (50,5 g, 0,193 mol) und Wasser (4,4 g, 0,245 mol) versetzt. Die Lösung wird 10 h bei Raumtemperatur gerührt und anschließend unter Rückfluß erhitzt. Das Lösemittel wird im Vakuum abgezogen. Der Rückstand wird in 500 ml Petrolether /Ether (3:1) aufgenommen, und Triphenylphosphinoxid wird abfiltriert. Das Lösemittel wird im Vakuum abgezogen, und der Rückstand in 480 ml Dichlormethan gelöst. Die Lösung wird mit Di-tert.butyldicarbonat (53,8 g, 0,247 mol) und Triethylamin (32,1 g, 0,317 mol) versetzt und 2 h bei Raumtemperatur gerührt. Das Lösemittel wird im Vakuum abgezogen, und der Rückstand an Kieselgel chromatographiert (Petrolether/Ether 5:1).
Ausbeute: 47,7 g (85% der Theorie)
$C_{17}H_{23}NO_4Si$ (343,3)
$^{1}$H-NMR ($CDCl_3$): $\delta$ = 0.03 (s, 6H), 0.84 (s, 9H), 1.37 (s, 9H), 5.76-5.93 (m, 2H)

Beispiel V

(2S,3R)-3-N-(tert.Butyloxycarbonyl)amino-2,3-dihydro-2-hydroxymethyl-6H-pyran

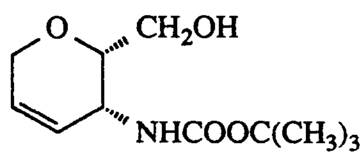

Eine Lösung der Verbindung aus Beispiel IV (45,0 g, 0,131 mol) und Tetrabutylammoniumfluorid (165 ml; eine 1,1 N Lösung in THF, 0,150 mol) wird 6 h bei Raumtemperatur gerührt. Das Solvens wird im Vakuum abgezogen und der Rückstand an Kieselgel chromatographiert (Petrolether/Essigester 1:1)
Ausbeute: 23,4g (78% der Theorie)
$C_{11}H_{19}NO_4$ (229,2)
$^1$H-NMR ($CDCl_3$): $\delta$ = 1.47 (s, 9H), 3.48-3.77 (m, 2H), 4.12 (cm, 1H), 4.20 (cm, 2H), 4.72 (d, J = 7Hz, 1H), 5.94 (cm, 2H)

Beispiel VI

(2S,3R)-3-(N-tert.Butyloxycarbonyl)amino-2,3-dihydro-6H-pyran-2-carbonsäure

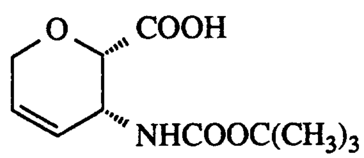

Eine Lösung der Verbindung aus Beispiel V (16,8 g, 73,4 mmol) in 20 ml Dichlormethan wird zu einer Lösung von Kaliumperoxodisulfat (52,6 g, 195 mmol) und Kaliumhydroxid (1,41 mol) in 1800 ml Wasser getropft. Die Mischung wird 20 h bei Raumtemperatur kräftig gerührt. Anschließend wird mit $Na_2S_2O_3$ x 5 $H_2O$ (58 g) versetzt, 90 min bei Raumtemperatur gerührt, mit NaCl (370 g) versetzt und 60 min bei Raumtemperatur gerührt. Die Reaktionsmischung wird über Celite filtriert und das Filtrat zweimal mit je 200 ml Ether extrahiert. Die Etherphasen werden über $Na_2SO_4$ getrocknet und im Vakuum eingeengt. Man erhält 1,7 g (10%) der Verbindung aus Beispiel V zurück. Die wäßrige Phase wird mit Essigsäure bis pH 1 angesäuert und zehnmal mit je 300 ml Essigester extrahiert. Die organische Phase wird über $Na_2SO_4$ getrocknet und das Solvens im Vakuum abgezogen. Der Rückstand wird ohne weitere Reinigung verwendet.
Ausbeute: 11,8 g (63% der Theorie)
$C_{11}H_{17}NO_5$ (243,2)
$^1$H-NMR ($CDCl_3$): $\delta$ = 1.42 (s, 9H), 4.15-4.64 (m, 4H), 5.0 (d, J = 9Hz, 1H), 5.94 (cm, 2H), 9.53 (s, 1H)

Beispiel VII

(2S,3R)-[3-(N-tert.Butyloxycarbonyl)amino-2,3-dihydro-6H-pyran-2-carbonsäuremethylester

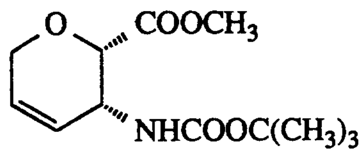

Eine Lösung der Verbindung aus Beispiel VI (6,50 g, 26,7 mmol) in 50 ml Ether wird mit Diazomethan (100 ml einer 4,1 N Losung in Ether) versetzt. Die Lösung wird 1 h bei Raumtemperatur gerührt, überschüssiges Diazomethan wird mit Essigsäure zerstört und das Solvens im Vakuum abgezogen. Der Rückstand wird an Kieselgel chromatographiert (Petrolether/Essigester 2:1)
Ausbeute: 4,90g (66% der Theorie)
$C_{12}H_{19}NO_5$ (257,2)
$[\alpha]_D^{20}$ = -155.2 (C = 0.31, $CHCl_3$)

Beispiel VIII

(2R,3S)-[3-(N-tert.Butyloxycarbonyl)amino-2,3-dihydro-6H-pyran-2-carbonsäuremethylester

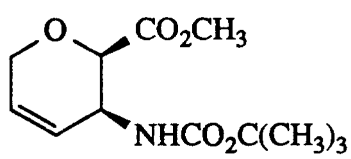

Die Titelverbindung wird in Analogie zur Vorschrift des Beispiels VII hergestellt.
$[\alpha]_D^{20}$ = +149 (c = 0.97, $CH_3OH$)

Herstellungsbeispiele

Beispiel 1

(2S,3R)-3-Amino-2,3-dihydro-6H-pyran-2-carbonsäuremethylester Hydrochlorid

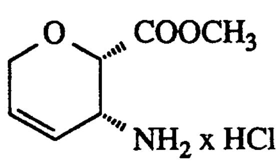

Eine Lösung der Verbindung aus Beispiel VII (3,80 g, 14,8 mmol) in 25 mi einer 4 N HCl-Lösung in Dioxan wird 2 h bei Raumtemperatur gerührt. 50 ml Ether werden zugetropft und der Niederschlag wird abgesaugt und getrocknet.
Ausbeute: 2,30g (80% der Theorie)
$C_7H_{11}NO_3$ x HCl
Smp.: 195 °C (Zers.)
$[\alpha]_D^{20}$ = -229 (c = 0.77, MeOH)

Beispiel 2 und Beispiel 3

(2S,3R)-3-(N-tert.Butyloxycarbonyl)amino-2,3-dihydro-6H-pyran-2-carbonsäure(tert.butylcarbonyloxy)-methylester

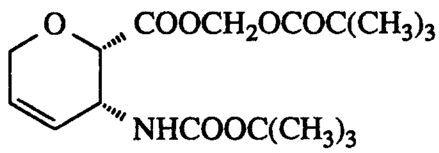

Beispiel 2

(2R,3R)-3-(N-tert.Butyloxycarbonyl)amino-2,3-dihydro-6H-pyran-2-carbonsäure(tert.butylcarbonyloxy)-methylester

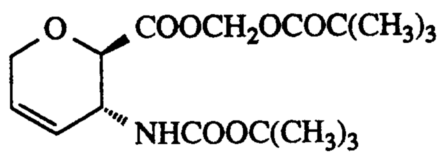

Beispiel 3

Eine Lösung von Natriumjodid (0,90 g, 6,0 mmol) und Pivalinsäurechlormethylester (0,45 g, 3,0 mmol) in 13 ml Aceton wird bei Raumtemperatur im Dunklen 48 h gerührt. Die Mischung wird filtriert, das Lösemittel im Vakuum abgezogen, und 10 ml THF werden zugegeben. Die Lösung wird bei -10 °C zu einer Lösung der Verbindung aus Beispiel VI (0,48 g, 2,0 mmol) und DBU (0,32 g, 2,1 mmol) in 10 ml THF zugetropft. Die Mischung wird 4 h bei 0 °C gerührt, mit 50 ml Wasser versetzt und dreimal mit je 30 ml Essigester extrahiert. Die vereinigten organischen Phasen werden mit 30 ml einer gesättigten $NaHCO_3$-Lösung und mit 30 ml NaCl-Lösung gewaschen. Die organische Phase wird über $Na_2SO_4$ getrocknet, das Solvens im Vakuum abgezogen und der Rückstand an Kieselgel chromatographiert (Petrolether/Essigester 2:1)
Ausbeute Beispiel 2: 0,131 g (18% der Theorie)
Ausbeute Beispiel 3: 0,064g (9% der Theorie)
$C_{17}H_{27}NO_7$ (357,3)

Beispiel 4 und Beispiel 5

(2S,3R)-3-(N-tert.Butyloxycarbonyl)amino-2,3-dihydro-6H-pyran-2-carbonsäure-tert.butylester

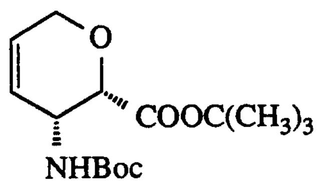

Beispiel 4

(2R,3R)-3-(N-tert.Butyloxycarbonyl)amino-2,3-dihydro-6H-pyran-2-carbonsäure-tert.butylester

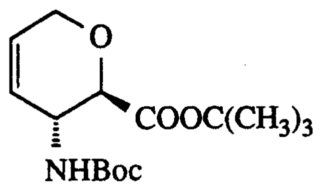

Beispiel 5

Eine Lösung der Verbindung aus Beispiel VI (0,30 g, 1,23 mmol), tert.Butanol (0,274 g, 3,70 mmol) und 4-Dimethylaminopyridin (0,015 g, 0,123 mmol) in 3 ml Dichlormethan wird bei 0 °C unter Argon mit einer Lösung von Dicyclohexylcarbodiimid (0,282 g, 1,36 mmol) versetzt. Die Mischung wird 2 h bei Raumtemperatur gerührt. Dicyclohexylharnstoff wird abfiltriert, das Filtrat zweimal mit je 2 ml 0,1 N HCl und zweimal mit gesättigter $NaHCO_3$-Lösung gewaschen, über $Na_2SO_4$ getrocknet und im Vakuum eingedampft. Der Rückstand wird an Kieselgel chromatographiert (Essigester/Petrolether 1:3)
Ausbeute Beispiel 4: 0,052 g (14% der Theorie)
Ausbeute Beispiel 5: 0,150 g (41% der Theorie)
$R_f$ für Beispiel 4: 0,41
$R_f$ für Beispiel 5: 0,33
$C_{15}H_{25}NO_5$ (299,4)
In Analogie zur Vorschrift der Beispiele 4 und 5 werden die in Tabelle 1 aufgeführten Beispiele hergestellt:

Tabelle 1:

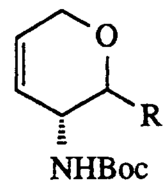

| Bsp.-Nr. | R | Fp. (°C) | $R_f$ (Petrolether/ Essigester 3:1) |
|---|---|---|---|
| 6 | $CO_2CH_2C_6H_5$ | 138 | 0.23 |
| 7 | $CO_2CH_2C_6H_5$ | | 0,30 |
| 8 | $CO_2CH(CH_3)_2$ | | 0,23 |
| 9 | $CO_2CH(CH_3)_2$ | | 0,30 |

Beispiel 10

(2S,3R)-[3-N-Benzoylamino-4,5-dihydro-6H-pyranyl]carbonsäure-methylester

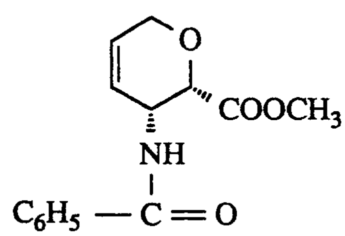

Eine Lösung der Verbindung aus Beispiel 1 (0,200 g, 1,03 mmol) in 1 ml Pyridin wird unter Eiskühlung mit Benzoylchlorid (0,145 g, 1,03 mmol) versetzt und 2 h bei Raumtemperatur gerührt. Anschließend werden 10 ml Dichlormethan zugetropft und zweimal mit je 10 ml 10%iger Salzsäure gewaschen. Die organische Phase wird über $Na_2SO_4$ getrocknet, das Solvens im Vakuum abgezogen, und der Rückstand an Kieselgel chromatographiert (Dichlormethan/Methanol 10:1)

Ausbeute: 0,193 g (72% der Theorie)

$C_{14}H_{15}NO_4$ (261,3)

$R_f$ = 0,29($CH_2Cl_2$/MeOH 10:1)

In Analogie zur Vorschrift des Beispiels 10 werden die in Tabelle 2 aufgeführten Beispiele hergestellt:

Tabelle 2:

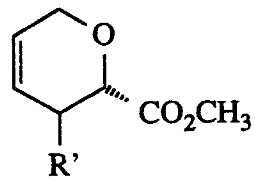

| Bsp.-Nr. | R' | $R_f$ |
|---|---|---|
| 11 | $NHCOCH_3$ | 0.33 [a)] |
| 12 | $NHCO_2CH_3$ | 0,45 [b)] |
| 13 | $NHCO_2CH_2C_6H_5$ | 0,52 [b)] |

a) $CH_2Cl_2$ / $CH_3OH$ (10:1)

b) $CH_2Cl_2$ / $CH_3OH$ (20:1)

Beispiel 14

3-(N-tert.-Butyloxycarbonyl)amino-6-(tert.-butyldimethyl)silyloxy-2,3-dihydro-6H-pyran-2-carbonsäure-p-nitrobenzylester

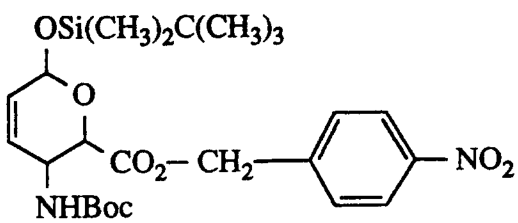

Zur Lösung von 1 Benzoyloxy-4-(tert.-butyloxycarbonyl)amino-1,3-butadien (5,0 g, 17,3 mmol) in 100 ml Tetrahydrofuran wird bei -10 °C eine 1,6 N Lösung von Methyllithium in Ether (32,4 ml, 51,8 mmol) getropft. Man läßt 1 h bei 0 °C rühren, tropft tert.-Butyldimethylsilylchlorid (5,40g, 36 mmol) in 20 ml THF hinzu, läßt 2 h bei 0 °C rühren, engt die Lösung i.Vak. auf ein Volumen von etwa 50 ml ein und versetzt mit 150 ml Benzol. Die Lösung wird einmal mit 50 ml einer 1 %igen wäßrigen $NH_4Cl$-Lösung und einmal mit 50 ml Wasser gewaschen. Die organische Phase wird mit $Na_2SO_4$ getrocknet, mit 100 mg Hydrochinon und mit einer Lösung von Glyoxylsäure-p-nitrobenzylester (5,10 g, 22,5 mmol) in 50 ml Benzol versetzt und 24 h am Wasserabscheider unter Rückfluß erhitzt. Das Lösungsmittel wird i.Vak. abgezogen und der Rückstand an Kieselgel chromatographiert (Petrolether/Essigester 2:1).
Ausbeute: 3,30 g (37 %) eines Diastereomerengemisches
$C_{24}H_{36}N_2O_4Si$ (508,6)
$R_f$ = 0.32 (Diastereomer A) und 0.40 (Diastereomer B), (Petrolether/Essigester 2:1)

Beispiel 15

3-(N-tert.-Butyloxycarbonyl)amino-6-(tert.-butyldimethyl)silyloxy-2,3-dihydro-6N-pyran-2-carbonsäuremethylester

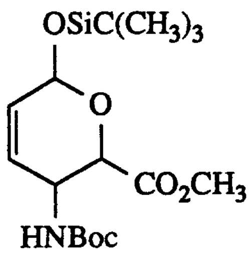

Zur Lösung der Verbindung aus Beispiel 14 (1,0 g, 2,0 mmol) in 20 ml Methanol wird bei 0 °C eine Lösung von Natriummethanolat (0,11 g, 2,0 mmol) in 10 ml Methanol getropft. Man läßt 15 min bei 0 °C rühren, neutralisiert mit DOWEX X50 W-X8, filtriert, zieht das Lösungsmittel i.Vak. ab und chromatographiert den Rückstand an Kieselgel (Petrolether/Essigester 2:1).
Ausbeute: 0,29 g (37 %) eines Diastereomerengemisches
$C_{18}H_{33}NO_6Si$ (387,5)
$R_f$ = 0.46 (Petrolether/Essigester 2:1)

Beispiel 16

(2S,3R)-3-Amino-2,3-dihydro-6H-pyran-2-carbonsäure-hydrochlorid

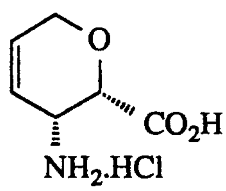

Eine Lösung der Verbindung aus Beispiel 1 (0,35 g, 1,80 mmol) in 30 ml 10%iger Salzsäure wird 2 h unter Rückfluß erhitzt. Die Lösung wird im Vakuum eingeengt und 5 h bei 50 °C/0.1 mbar getrocknet.
Ausbeute: 0,75 g (100%)
$C_6H_9NO_3$ x HCl (143.2 x 36.5)
Smp. 210 °C(Zers.), $[\alpha]_D^{20}$ = -242.3 (c = 1.00, $CH_3OH$)

Beispiel 17

(2R,3S)-3-Amino-2,3-dihydro-6H-pyran-2-carbonsäure-hydrochlorid

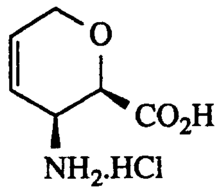

Die Titelverbindung wird in Analogie zur Vorschrift des Beispiels 16 aus (2R,3S)-3-Amino-2,3-dihydro-6H-pyran-2-carbonsäuremethylester hergestellt.
Smp. 210 ° C(Zers.), $[\alpha]_D^{20}$ = +241.8 (c = 1.00, $CH_3OH$)

Beispiel 18

(2R,3S)-3-Amino-2,3-dihydro-6H-pyran-2-carbonsäuremethylester-hydrochlorid

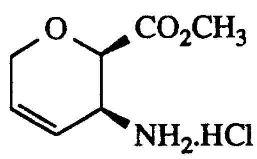

In Analogie zur Vorschrift des Beispiels 1 wird die Titelverbindung hergestellt. Smp. 192 ° C(Zers.), $[\alpha]_D^{20}$ = +221,2 (c = 1.00, $CH_3OH$)

**Patentansprüche**

**1.** Substituierte 2,3-Dihydropyrane der allgemeinen Formel (I)

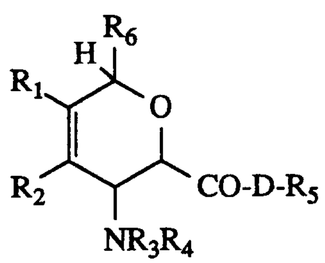

(I),

in welcher

| | |
|---|---|
| $R^6$ | für Wasserstoff oder geradkettiges oder verzweigtes Alkyl mit bis zu 8 Kohlenstoffatomen steht, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist, oder für eine Gruppe der Formel $-OSi(R)_3$ steht, worin |
| R | Methyl, Isopropyl, tert.-Butyl oder Phenyl bedeutet, |
| $R^1$ und $R^2$ | gleich oder verschieden sind und |

für Wasserstoff oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen substituiert ist,

$R^3$ für eine Aminoschutzgruppe,
für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl oder Alkoxycarbonyl mit jeweils bis zu 8 Kohlenstoffatomen, oder
für Phenoxy oder Benzoyl steht, das gegebenenfalls wie oben beschrieben substituiert ist, oder
für eine Gruppe der Formel $-SO_2R^7$ steht,
worin

$R^7$ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^8R^9$ substitituiert sind,
worin

$R^8$ und $R^9$ gleich oder verschieden sind und Wasserstoff, geradkettiges oder verzweigtes Alkyl mit bis zu 8 Kohlenstoffatomen oder Phenyl bedeuten,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-SO_2R^7$ oder $-NR^8R^9$ substituiert ist,
worin

$R^7$, $R^8$ und $R^9$ die oben angegebene Bedeutung haben,

$R^4$ für Wasserstoff oder für geradkettiges oder verzweigtes Alkyl mit bis zu 8 Kohlenstoffatomen steht, das gegebenenfalls durch Phenyl substituiert ist,

oder

$R^3$ und $R^4$ gemeinsam für den Rest der Formel $=CHR^{6'}$ stehen,
worin

$R^{6'}$ die oben angegebene Bedeutung von $R^6$ hat und mit dieser gleich oder verschieden ist,

D für ein Sauerstoff- oder Schwefelatom oder für die $>$NH-Gruppe steht,

$R^5$ für Wasserstoff oder für geradkettiges oder verzweigtes Alkyl mit bis zu 8 Kohlenstoffatomen, Benzyl oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-SO_2R^7$ oder $-NR^8R^9$ substituiert sind,
worin

$R^7$, $R^8$ und $R^9$ die oben angegebene Bedeutung haben,
oder
für geradkettiges oder verzweigtes Alkoxy mit bis zu 8 Kohlenstoffatomen steht, das gegebenenfalls durch geradkettiges oder verzweigtes Alkoxycarbonyl mit bis zu 6 Kohlenstoffatomen substituiert ist,

oder für den Fall, daß D für die $>$NH-Gruppe steht,

$R^5$ für die Gruppe der Formel $-SO_2R^7$ steht,
worin
$R^7$ die oben angegebene Bedeutung hat.

**2.** Substituierte 2,3-Dihydropyrane gemäß Anspruch 1, in welcher

$R^6$ für Wasserstoff oder geradkettiges oder verzweigtes Alkyl mit bis zu 6 Kohlenstoffatomen steht, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist, oder
für eine Gruppe der Formel $-OSi(R)_3$ steht,
worin

R Methyl, Isopropyl, tert.-Butyl oder Phenyl bedeutet,

$R^1$ und $R^2$ gleich oder verschieden sind und
für Wasserstoff oder für geradkettiges oder verzweigtes Alkyl mit bis zu 6 Kohlenstoffatomen stehen, das gegebenenfalls durch Halogen, Hydroxy, durch geradkettiges oder verzweigtes Alkoxy, Acyl oder Alkoxycarbonyl mit jeweils bis zu 4 Kohlenstoffatomen substituiert ist,

$R^3$ für tert.Butyloxycarbonyl (BOC), Benzyloxycarbonyl (Z) oder Phenoxycarbonyl steht, oder
für Wasserstoff oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl oder Alkoxycarbonyl mit jeweils bis zu 6 Kohlenstoffatomen oder für Phenoxy oder Benzoyl steht, das gegebenenfalls wie oben beschrieben substituiert ist, oder für eine Gruppe der Formel $-SO_2R^7$ steht,
worin

$R^7$ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^8R^9$ substituiert ist,
worin

$R^8$ und $R^9$ gleich oder verschieden sind und Wasserstoff, geradkettiges oder verzweigtes Alkyl mit bis zu 6 Kohlenstoffatomen bedeuten,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-SO_2R^7$ oder $-NR^8R^9$ substituiert ist,
worin

$R^7$, $R^8$ und $R^9$ die oben angegebene Bedeutung haben,

$R^4$ für Wasserstoff oder für geradkettiges oder verzweigtes Alkyl mit bis zu 6 Kohlenstoffatomen oder Benzyl steht,

oder

$R^3$ und $R^4$ gemeinsam für den Rest der Formel $=CHR^{6'}$ stehen,
worin

$R^{6'}$ die oben angegebene Bedeutung von $R^5$ hat und mit dieser gleich oder verschieden ist,

D für ein Sauerstoff- oder Schwefelatom oder für die $>$NH-Gruppe steht,

$R^5$ für Wasserstoff oder für geradkettiges oder verzweigtes Alkyl mit bis zu 6 Kohlenstoffatomen, Benzyl oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe

der Formel $-SO_2R^7$ oder $-NR^8R^9$ substituiert sind,
worin
$R^7$, $R^8$ und $R^9$ die oben angegebene Bedeutung haben,
oder
für geradkettiges oder verzweigtes Alkoxy mit bis zu 6 Kohlenstoffatomen steht, das gegebenenfalls durch geradkettiges oder verzweigtes Alkoxycarbonyl mit bis zu 4 Kohlenstoffatomen substituiert ist,

oder für den Fall, daß D für die >NH-Gruppe steht,
$R^5$ für die Gruppe der Formel $-SO_2R^7$ steht,
worin
$R^7$ die oben angegebene Bedeutung hat.

**3.** Substituierte 2,3-Dihydropyrane gemäß Anspruch 1, in welcher
$R^6$ für Wasserstoff oder geradkettiges oder verzweigtes Alkyl mit bis zu 4 Kohlenstoffatomen steht, oder
für eine Gruppe der Formel $-OSi(R)_3$ steht,
worin
R Methyl, Isopropyl, tert.-Butyl oder Phenyl bedeutet,
$R^1$ und $R^2$ gleich oder verschieden sind und
für Wasserstoff oder
für geradkettiges oder verzweigtes Alkyl mit bis zu 4 Kohlenstoffatomen stehen,
$R^3$ für tert.Butyloxycarbonyl (Boc), Benzyloxycarbonyl (Z) oder Phenoxycarbonyl steht, oder
für Wasserstoff steht, oder
für geradkettiges oder verzweigtes Alkyl mit bis zu 4 Kohlenstoffatomen steht, oder
für geradkettiges oder verzweigtes Acyl oder Alkoxycarbonyl mit jeweils bis zu 4 Kohlenstoffatomen oder für Phenoxy oder Benzoyl steht, oder
für eine Gruppe der Formel $-SO_2R^7$ steht,
worin
$R^7$ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
oder
für geradkettiges oder verzweigtes Alkoxy mit bis zu 4 Kohlenstoffatomen steht, das gegebenenfalls durch geradkettiges oder verzweigtes Alkoxycarbonyl mit bis zu 4 Kohlenstoffatomen substituiert ist,
$R^4$ für Wasserstoff oder
für geradkettiges oder verzweigtes Alkyl mit bis zu 4 Kohlenstoffatomen steht,

oder
$R^3$ und $R^4$ gemeinsam für den Rest der Formel $=CHR^{6'}$ stehen,
worin
$R^{6'}$ die oben angegebene Bedeutung von $R^6$ hat und mit dieser gleich oder verschieden ist,
D für ein Sauerstoff- oder ein Schwefelatom oder für die >NH-Gruppe steht,
$R^5$ für Wasserstoff oder für geradkettiges oder verzweigtes Alkyl mit bis zu 4 Kohlenstoffatomen Benzyl oder Phenyl steht, wobei letztere gegebenenfalls durch Fluor, Chlor, Brom, Nitro, Cyano, Methoxy oder Ethoxy oder durch eine Gruppe der Formel $-SO_2R^7$ oder $-NR^8R^9$ substituiert sind,
worin
$R^7$ die oben angegebene Bedeutung hat
und
$R^8$ und $R^9$ gleich oder verschieden sind und Wasserstoff, Methyl oder Ethyl bedeuten

oder im Fall, daß D für die >NH-Gruppe steht,
$R^5$ für die Gruppe der Formel $-SO_2R^7$ steht,
worin
$R^7$ die oben angegebene Bedeutung hat.

4. Verfahren zur Herstellung der Verbindungen der allgemeinen Formel (I) gemäß Anspruch 1, dadurch gekennzeichnet, daß man [A] Verbindungen der allgemeinen Formel (II)

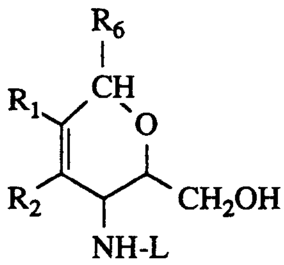

(II),

in welcher

$R^1$, $R^2$ und $R^6$ die oben angegebene Bedeutung haben,

und

L für eine der oben aufgeführten Aminoschutzgruppen, vorzugsweise für tert.Butyloxycarbonyl (Boc), steht,

in Anwesenheit von Oxidationsmitteln, gegebenenfalls in inerten Lösemitteln zunächst zu den Verbindungen der allgemeinen Formel (III)

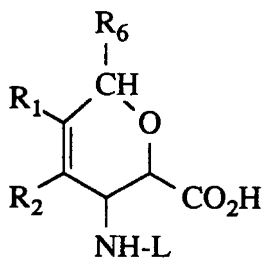

(III),

in welcher

$R^1$, $R^2$, $R^6$ und L die oben angegebene Bedeutung haben,

oxidiert

und anschließend gegebenenfalls die Säuren in inerten Lösemitteln entweder direkt, beispielsweise mit Diazomethan oder nach vorgeschalteter Aktivierung, gegebenenfalls in Anwesenheit von Hilfsmitteln und/oder einer Base, verestert und gegebenenfalls unter Freisetzung der Aminfunktion ($R^3$,$R^4$ = H) die Aminoschutzgruppe L nach üblichen Methoden, vorzugsweise mit Säuren, zunächst abspaltet, und im Fall, der für $R^3$, $R^4$, D und $R^5$ oben aufgeführten anderen Definitioen, nach üblichen Verfahren, wie beispielsweise Amidierung, Sulfonierung oder Sulfoamidierung, gegebenenfalls in Anwesenheit von Hilfsstoffen, wie Katalysatoren und/oder Dehydratisierungsmitteln, derivatisiert, oder daß man

[B] Verbindungen der allgemeinen Formel (IV)

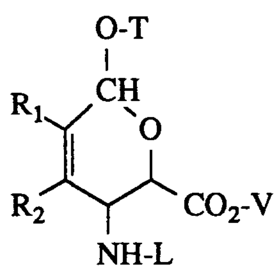

(IV),

in welcher

$R^1$, $R^2$ und L die oben angegebene Bedeutung haben,

T für eine typische Hydroxygruppe, vorzugsweise für tert.-Butyldimethylsilyl, steht,
und
V für eine Schutzgruppe, vorzugsweise p-Nitrobenzyl, steht,
umsetzt,
in einem nächsten Schritt die Schutzgruppe (V) in inerten Lösemitteln nach üblicher Methode, vorzugsweise mit Natriummethanolat, abspaltet, anschließend die Gruppe O-T durch Umsetzung mit Acetanhydrid, n-$Bu_4N^{\oplus}F^{\ominus}$ in Pyridin in die -OCO-$CH_3$-Gruppe überführt und in einem letzten Schritt, ebenfalls nach üblicher Methode, vorzugsweise mit Triethylsilan in Anwesenheit von Trimethylsilyltriflat die Gruppe -O-T abspaltet, und im Fall, daß $R^6 \neq H$ eine Alkylierung anschließt,
oder gegebenenfalls Derivatisierungen sowohl an der Ester- als auch an der Aminfunktion, wie unter [A] beschrieben, durchführt,
und im Fall der Säuren (D = O, $R^5$ = H) die entsprechenden Ester verseift.

**5.** Verwendung der Verbindungen der allgemeinen Formel (I) gemäß Anspruch 1 zur Bekämpfung von Krankheiten.

**6.** Arzneimittel enthaltend Verbindungen der allgemeinen Formel (I) gemäß Anspruch 1.

**7.** Verwendung der Verbindungen der allgemeinen Formel (I) gemäß Anspruch 1 zur Herstellung von Arzneimitteln in der Human- oder Veterinärmedizin.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 7260

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| D | TETRAHEDRON LETTERS, Bd. 24, Nr. 43, Oktober 1983, Oxford, GB, Seiten 4661 - 4664 R.R. SCHMIDT, ET AL: 'Synthesis fo a uracinine derivative via hetero Diels-Alder reaction' | | C07D309/28 C07F7/18 A61K31/35 |
| X | * Verbindungen 11a, 11b, 12a * | 1 | |
| A | * Verbindung 1 * --- | 1,5-7 | |
| P,A | EP-A-0 482 410 (BAYER) * Seite 2 * ----- | 1,5-7 | |
| | | | RECHERCHIERTE SACHGEBIETE (Int. Cl. 5) |
| | | | C07D |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 24 NOVEMBER 1992 | RUSSELL F. ENGLISH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

EP 92 11 7260

-C-

Bemerkung: Obwohl der Anspruch 5
sich auf ein Verfahren zur Behandlung des
menschlichen/tierischen Körpers
(Diagnostizierverfahren, das am menschlichen/
tierischen Körper vorgenommen wird,)
bezieht (Art. 52(4)EPU), wurde die
Recherche durchgeführt und gründete sich auf
die angeführten Wirkungen der Verbindung/
Zusammensetzung.